# EUROPEAN PATENT SPECIFICATION

(11) **EP 2 083 759 B1**
(45) Date of publication and mention of the grant of the patent: **02.09.2015**
(21) Application number: 07824202.1
(22) Date of filing: 17.10.2007
(51) Int. Cl.: A61F 2/40

(54) **SHOULDER PROSTHESIS**
SCHULTERPROTHESE
PROTHÈSE D'ÉPAULE

(30) Priority: 23.10.2006 GB 0621061
(43) Date of publication of application: 05.08.2009
(73) Proprietor: Innovative Design Orthopaedics Limited, London, W1U 7GB (GB)
(72) Inventor: LEVY, Ofer, Henley on Thames Oxon RG9 5HS (GB); COPELAND, Stephen, Henley on Thames Oxfordshire RG9 4AA (GB); SMIRTHWAITE, Paul, Bath BA2 4PS (GB)
(74) Representative: Straus, Alexander
(86) International application number: PCT/GB2007/003952
(87) International publication number: WO 2008/050091

(56) References cited:
- EP-A- 1 520 560
- EP-A- 1 649 836
- WO-A-2005/032430
- WO-A-2007/031575
- WO-A-2007/082925
- WO-A-2007/084939
- FR-A- 2 855 743
- US-A- 3 978 528

## Description

This invention relates to a shoulder prosthesis and particularly but not exclusively relates to a bearing component for use with a humeral prosthesis.

### BACKGROUND

In the field of reconstructive joint surgery, it is known to replace a deficient shoulder joint with a total shoulder prosthesis. In cases of severe damage to the rotator cuff, it is known to employ a "reverse" type shoulder prosthesis, in which the anatomy of a healthy shoulder is reversed, with a spherical ball component being implanted in the glenoid cavity of the scapula, and a cup component being implanted in a proximal end of the humerus. The design of the reverse shoulder prosthesis enables the upward force provided by the deltoid muscle to be employed for the purposes of rotation, thus compensating, at least in part, for a deficient rotator cuff and restoring some measure of abduction to a patient's arm. In order to recruit as many deltoid fibres as possible for the purposes of abduction, and to reduce torque at the point of fixation of the glenoid component, the centre of rotation of the prosthetic joint is designed to be in a more medial position than that of a normal, healthy joint. This medialisation of the centre of rotation is achieved through the design of the glenoid component, which is a large diameter partial sphere having no neck. Medialisation of the centre of rotation, and consequently of the humerus, leads to detensioning of the deltoid. To address this, and to improve the power of the deltoid, the humeral cup is oriented with a non anatomic inclination of approximately 155°, thus lowering the humerus and over tensioning the deltoid muscle.

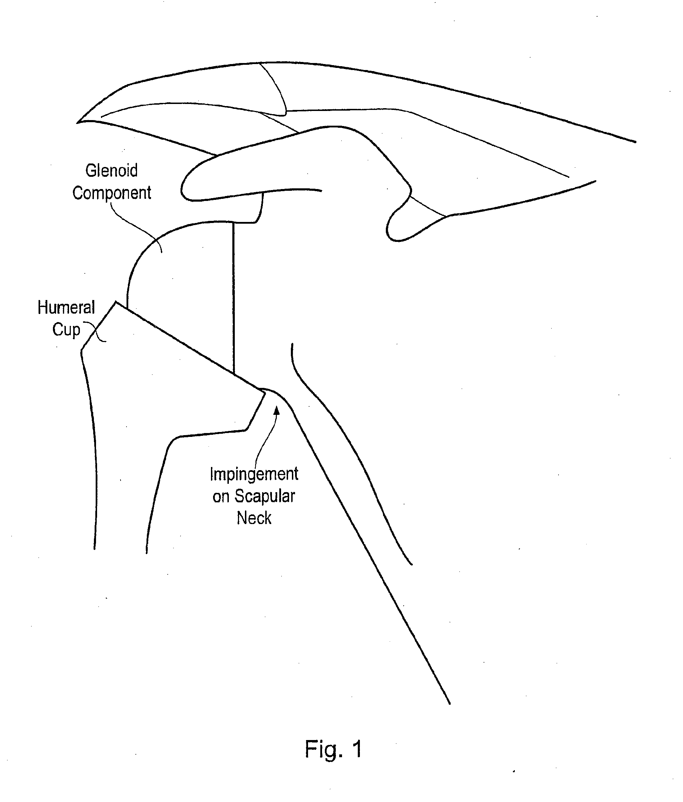

Medialisation of the centre of rotation of the joint and lowering of the humerus are essential biomechanical features of a reverse shoulder prosthesis. However, the inevitable consequence of these features is impingement of the medial aspect of the humeral cup on the inferior scapular neck with the arm in an adducted position (as illustrated in Figure 1). This impingement damages the humeral cup and causes scapular notching, which can be an initiating factor for osteolysis. Further bone degradation may be triggered by wear particles from the humeral cup. Osteolysis in this region can result in loosening of the glenoid component. In addition, impingement of the humeral cup on the inferior scapular neck can cause superior (asymmetric) decoaptation, where a superior portion of the humeral cup loses contact with the glenoid component. Such decoaptation reduces prosthetic stability.

US 3 978 528 A discloses a humeral component of a reverse shoulder prosthesis comprising a cup with a rim, a split pair of insert liners and a lock ring to be attached to the rim, whereby both, the rim and the lock ring, have a uniform thickness about their circumference.

### SUMMARY OF THE INVENTION

According to the present invention, there is provided a bearing component for use in a humeral prosthesis, the bearing component comprising a concave bearing surface that terminates at a continuous substantially circular rim, the rim defining a first plane; a convex engaging surface, that is on an opposite side of the bearing component to the bearing surface and terminates in an annular shoulder, the shoulder defining a second plane; and a connecting region which extends between the first and second planes, wherein the first and second planes are not parallel, wherein the bearing component together with a stem is configured to form a humeral part of a reverse shoulder prosthesis. In an embodiment of the invention, the rim of the bearing component is angled with respect to the base of the bearing component, causing the thickness of the bearing component to vary about its circumference. Such variation in thickness can be employed to advantage when the bearing component is implanted in a patient. For example, the bearing component may be implanted with the area of least thickness in an inferior, medial position. In this orientation, projection of the bearing component towards the inferior scapular neck is reduced, thus reducing the occurrence of scapular impingement and notching.

The concave bearing surface may comprise less than a hemisphere. It is an advantage of the invention that the reduced area of the bearing surface affords a greater range of motion to a prosthetic joint in which the bearing component of the present invention is employed, thus assisting to reduce the occurrence of scapular impingement and notching. It has been discovered that the reduction in contact area between the bearing component and the prosthesis component against which it articulates need not lead to a lessening of the stability of the joint.

An angle between the first and second planes of the bearing component may be between 5 and 20 degrees but is preferably approximately 10 degrees. The angle may be selected to provide the optimum variation in thickness about the circumference of the bearing component.

The connecting region may comprise a connecting wall, which may be substantially in the shape of a truncated cylinder.

The annular shoulder of the convex engaging surface may define an annular bearing surface. The annular shoulder may comprise at least one recess.

The bearing component may be formed from a polymeric material. The polymeric material may be ultra high molecular weight polyethylene. Alternatively, the polymeric material may be any suitable biologically compatible polymeric material.

The bearing component may further comprise a lining component. The lining component may comprise a metallic shell that lines the concave bearing surface of the bearing component. In this manner, the bearing component of the present invention may be employed in a metal-on-metal articulation.

According to another aspect of the present invention, there is provided a humeral prosthesis comprising a bearing component as disclosed above, and a stem.

The engaging surface of the bearing component may be received within the stem such that the annular bearing surface of the bearing component engages a corresponding annular bearing surface on the stem.

The annular bearing surface of the stem may comprise at least one projecting lug, adapted to be received within the recess on the annular shoulder of the bearing component.

The annular shoulder of the bearing component may comprise a plurality of recesses arranged circumferentially around the bearing component. Similarly, the annular bearing surface of the stem may comprise a plurality of projecting lugs, adapted to be received within the plurality of recesses on the annular shoulder of the bearing component.

The corresponding recesses and lugs therefore form an indexing system. It is an advantage of the humeral prosthesis of the present invention that the bearing component may be securely inserted into the stem at any desired rotational orientation. Further, the relative rotational orientation of the two components may be altered simply and easily. For example, when implanting the prosthesis into a patient, the surgeon may orientate the bearing component such that the portion having the least thickness is placed at the position where the anatomy of the patient dictates scapular impingement is most likely to occur. If a trial reduction suggests the bearing component has not been inserted at the optimal position, this may be corrected, simply by repositioning the bearing component.

According to another aspect of the present invention, there is provided a shoulder prosthesis comprising a humeral prosthesis as claimed in any one of claims 10 to 14 and a glenoid prosthesis. The glenoid prosthesis may comprise a glenoid screw (metaglene) and a glenoid head (glenosphere).

### BRIEF DESCRIPTION OF THE DRAWINGS

For a better understanding of the present invention, and to show more clearly how it may be carried into effect, reference will now be made, by way of example, to the accompanying drawings, in which:-
Figure 1 is a side view illustrating scapular notching in a reverse shoulder prosthesis,
Figure 2 is a side view of a humeral bearing component,
Figure 3 is a perspective view of a humeral stem and bearing component,
Figure 4 is a side view of an assembled reverse shoulder prosthesis
Figure 5 is an illustrative view of an implanted reverse shoulder prosthesis.

### DETAILED DESCRIPTION OF PREFERRED EMBODIMENTS

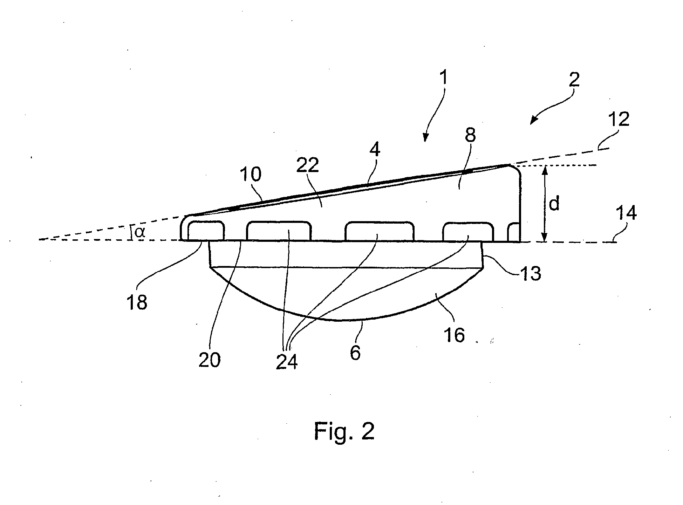

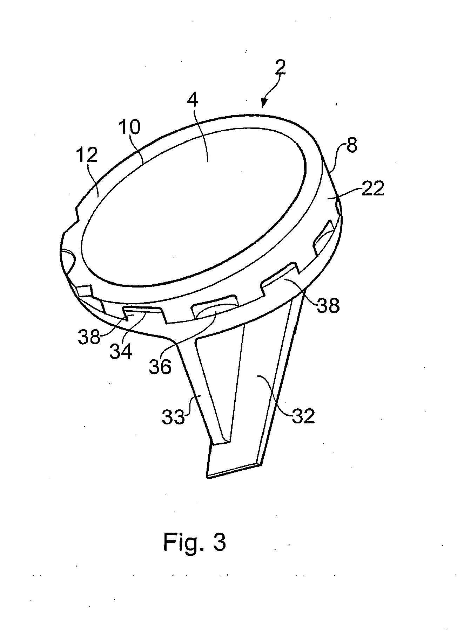

With reference to Figures 2 and 3, a humeral bearing component 2 comprises a substantially cup shaped body 1, having a concave bearing surface 4, a convex engaging surface 6 and a connecting region 8. The bearing surface 4 is substantially spherical and terminates at a substantially circular rim 10, that defines a first plane 12. The engaging surface 6 comprises a cylindrical part 13 and a spherical part 16 and is designed to form one half of a standard taper connection. The engaging surface 6 terminates at a second plane 14. The connecting region 8 extends from the first plane 12 to the second plane 14 and terminates at the second plane 14 in an annular shoulder 18 that forms an annular bearing surface 20. The first and second planes 12 and 14 intersect at an angle α such that the depth d of the connecting region 8 varies about the circumference of the bearing component 2. The angle α may be between 5 and 20 degrees but is preferably approximately 10 degrees.

The connecting region 8 is in the form of a substantially cylindrical wall 22. The wall 22 includes a plurality of recesses 24 that extend into the wall 22 from the annular bearing surface 20. Those recesses 24 that are located at the part of the wall 22 having the least depth d may extend through the entire depth d of the wall 22.

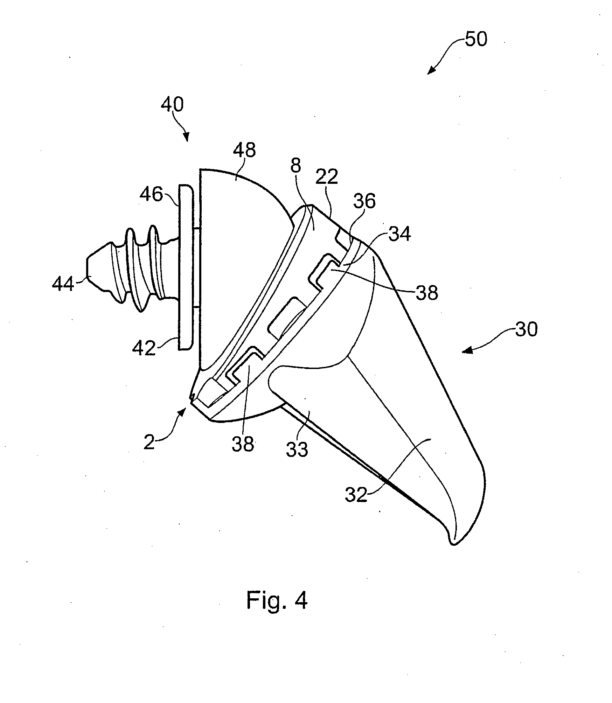

With reference also to Figure 4, the humeral bearing component 2 is suitable for use with a humeral stem 30. The stem 30 comprises a flange in the form of a fin 32 and a socket portion 34. The socket portion 34 is connected to the fin 32 by a web 33 that imparts rotational stability to the stem 30. The socket portion 34 is shaped to receive the engaging surface 6 of the bearing component 2 and includes an annular bearing surface 36. When the bearing component 2 is received within the stem 30, the annular bearing surface 20 of the bearing component 2 is in contact with the annular bearing surface 36 of the stem 30. The socket portion 34 of the stem 30 includes a plurality of lugs 38 that protrude from the annular bearing surface 36 on the stem 30. The lugs 38 are shaped to be received within the recesses 24 on the bearing component 2. The lugs 38 and recesses 24 form an indexing system, preventing relative rotational movement between the bearing component 2 and the stem 30 when assembled, but permitting the bearing component 2 to be received within the stem 30 at a plurality of rotational orientations.

The bearing component 2 is formed from a polymeric material which may be polyethylene, ultra high molecular weight polyethylene (UHMWPE) or any other suitable, biocompatible polymeric material. The bearing component 2 may also include a metallic liner (not shown). The liner may cover substantially the entire bearing surface 4 so as to provide a metallic bearing surface, if required. The stem 30 is made from a metallic material which may be titanium, cobalt chrome or any other suitable biocompatible metallic material including stainless steel and tantalum. The stem is preferably made from cobalt chrome. The stem may be coated with a porous metal, with Hydroxyapatite, or with any other material suitable for supporting bone ingrowth.

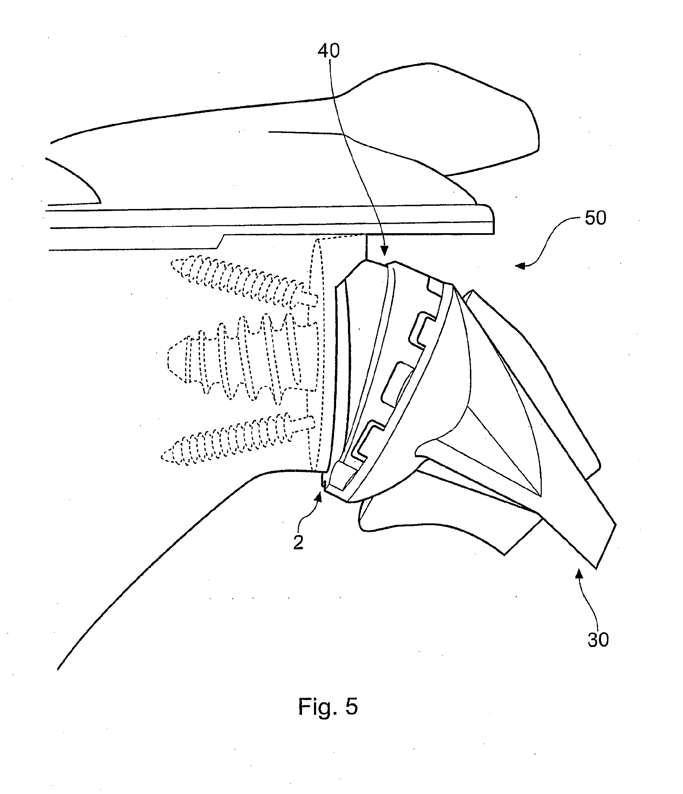

Referring to Figures 4 and 5, the humeral bearing 2 and stem 30 together form the humeral part of a reverse shoulder prosthesis 50. The glenoid part 40 of the prosthesis 50 comprises a metal plate, or glenoid screw (metaglene) 42, having a threaded protrusion 44 extending from a bone engaging face 46 thereof, and a glenoid head (glenosphere) 48. The glenoid head (glenosphere) 48 comprises at least a portion of a sphere and is mounted on the glenoid screw (metaglene) 42. When assembled, the glenoid and humeral parts of the prosthesis 50 articulate, with at least a portion of the glenoid head (glenosphere) 48 being received within the bearing component 2.

In use, the prosthesis 50 is implanted within a patient's shoulder and a trial reduction is performed to asses the range of motion afforded by the prosthesis. The limiting feature in adduction is, as explained above, impingement of the inferior medial aspect of the humeral bearing component 2 on the inferior scapular neck. By orienting the bearing component 2 such that the thinnest section of the bearing component is in the inferior medial position, the range of motion that can be achieved before impingement occurs is increased. In some patents, impingement may occur in a slightly more anterior or more posterior position than is usual. In such cases, the indexing system of the humeral prosthesis enables the bearing component to be rotated, so as to ensure that the thinnest section of the bearing component 2 is positioned at the point of impingement, thus gaining the greatest increase in range of motion from the design of the bearing component.

## Claims

1. A bearing component (2) for use in a humeral prosthesis, the bearing component comprising
a concave bearing surface (4) that terminates at a continuous substantially circular rim (10), the rim defining a first plane (12);
a convex engaging surface (6), that is on an opposite side of the bearing component to the bearing surface and terminates in an annular shoulder (18), the shoulder defining a second plane (14); and
a connecting region (8) which extends between the first and second planes, wherein the first and second planes are not parallel,
wherein the bearing component together with a stem (30) is configured to form a humeral part of a reverse shoulder prosthesis.

2. A bearing component (2) as claimed in claim 1 wherein the concave bearing surface (4) comprises less than a hemisphere.

3. A bearing component (2) as claimed in claim 1 or 2, wherein an angle between the first and second planes is between 5 and 20 degrees.

4. A bearing component (2) as claimed in any of the preceding claims, wherein the connecting region (8) comprises a connecting wall (22), substantially in the shape of a truncated cylinder.

5. A bearing component (2) as claimed in any one of the preceding claims, wherein the annular shoulder (18) defines an annular bearing surface (20).

6. A bearing component as claimed in any one of the preceding claims, wherein the annular shoulder comprises at least one recess.

7. A bearing component (2) as claimed in any one of the preceding claims, wherein the bearing component is formed from a polymeric material.

8. A bearing component (2) as claimed in claim 7, wherein the polymeric material is ultra high molecular weight polyethylene.

9. A bearing component (2) as claimed in any one of the preceding claims, further comprising a lining component.

10. A bearing component (2) as claimed in claim 9, wherein the lining component comprises a metallic shell that lines the concave bearing surface (4) of the bearing component.

11. A humeral prosthesis comprising a bearing component (2) as claimed in any one of the proceeding claims and a stem (30).

12. A humeral prosthesis as claimed in claim 11, wherein the engaging surface (6) of the bearing component (2) is received within the stem (30) such that the annular bearing surface (20) of the bearing component engages a corresponding annular bearing surface (36) on the stem.

13. A humeral prosthesis as claimed in claim 11 or 12, wherein the annular bearing surface (36) of the stem (30) comprises at least one projecting lug (38), adapted to be received within the recesses (24) on the annular shoulder (18) of the bearing component (2).

14. A humeral prosthesis as claimed in any one of claims 11 to 13, wherein the annular shoulder of the bearing component comprises a plurality of recesses arranged circumferentially around the bearing component.

15. A humeral prosthesis as claimed in claim 14, wherein the annular bearing surface (36) of the stem (30) comprises a plurality of projecting lugs (38), adapted to be received within the plurality of recesses (24) on the annular shoulder (18) of the bearing component (2).

16. A shoulder prosthesis (50) comprising a humeral prosthesis as claimed in any one of claims 11 to 15 and a glenoid prosthesis (40).

17. A shoulder prosthesis (50) as claimed in claim 16, wherein the glenoid prosthesis (40) comprises a glenoid screw (42) and a glenoid head (48).

## Patentansprüche

1. Tragelement (2) zur Verwendung in einer Humerusprothese, wobei das Tragelement (2) umfasst:
Eine konkave Tragoberfläche (4), die an einem durchgehenden, im Wesentlichen kreisförmigen Kranz (10) endet, wobei der Kranz eine erste Fläche (12) definiert,
eine konvexe Eingriffoberfläche (6), die an dem Trageelement gegenständig der Tragoberfläche angeordnet ist und in einer ringförmigen Schulter (18) endet, wobei die Schulter eine zweite Fläche (14) definiert, und
einen Verbindungsbereich (8), der sich zwischen der ersten und der zweiten Fläche erstreckt, wobei die erste und die zweite Fläche nicht parallel zueinander liegen,
wobei das Tragelement zusammen mit einem Schaft (30) angepasst ist, den humeralen Teil einer inversen Schulterprothese zu bilden.

2. Tragelement (2) nach Anspruch 1, wobei die konkave Tragoberfläche (4) weniger umfasst als eine Halbkugel.

3. Tragelement (2) nach Anspruch 1 oder 2, wobei der Winkel zwischen der ersten und der zweiten Fläche zwischen 5 und 20° beträgt.

4. Tragelement (2) nach einem der vorstehenden Ansprüche, wobei der Verbindungsbereich (8) eine Verbindungswand (22) umfasst, die im Wesentlichen die Form eines verkürzten Zylinders aufweist.

5. Tragelement (2) nach einem der vorstehenden Ansprüche, wobei die ringförmige Schulter (18) eine ringförmige Tragoberfläche (20) definiert.

6. Tragelement (2) nach einem der vorstehenden Ansprüche, wobei die ringförmige Schulter wenigstens eine Vertiefung umfasst.

7. Tragelement (2) nach einem der vorstehenden Ansprüche, wobei das Tragelement aus einem polymeren Material gefertigt ist.

8. Tragelement (2) nach Anspruch 7, wobei das polymere Material ein ultrahochmolekulargewichtiges Polyethylen ist.

9. Tragelement (2) nach einem der vorstehenden Ansprüche, ferner umfassend eine Auskleidungskomponente.

10. Tragelement (2) nach Anspruch 9, wobei die Auskleidungskomponente eine metallene Hülle umfasst, die die konkave Tragoberfläche (4) des Tragelements auskleidet.

11. Humeralprothese, umfassend ein Tragelement (2) nach einem der vorstehenden Ansprüche und einen Schaft (30).

12. Humeralprothese nach Anspruch 11, wobei die Eingriffoberfläche (6) des Tragelements (2) in dem Schaft (30) derart ausgenommen ist, dass die ringförmige Tragoberfläche (20) des Tragelements in Eingriff mit einer entsprechenden ringförmigen Tragoberfläche (36) auf dem Schaft (30) kommt.

13. Humeralprothese nach Anspruch 11 oder 12, wobei die ringförmige Tragoberfläche (36) des Schafts (30) wenigstens einen vorstehenden Ansatz (38) umfasst, der angepasst ist, in der Vertiefung (24) der ringförmigen Schulter (18) des Tragelements (2) aufgenommen zu werden.

14. Humeralprothese nach Anspruch 11 bis 13, wobei die ringförmige Schulter des Tragelements mehrere Vertiefungen umfasst, die umlaufend um das Tragelement angeordnet sind.

15. Humeralprothese nach Anspruch 14, wobei die ringförmige Tragoberfläche (36) des Schafts (30) mehrere vorstehende Ansätze (38) umfasst, die angepasst sind, in den mehreren Vertiefungen (24) der ringförmigen Schulter (18) des Tragelements (2) aufgenommen zu werden.

16. Schulterprothese (50) umfassend eine Humeralprothese nach einem der Ansprüche 11-15 und eine Glenoidprothese (40).

17. Schulterprothese (50) nach Anspruch 16, wobei die Glenoidprothese (40) eine Glenoidschraube (42) und einen Glenoidkopf (48) umfasst.

## Revendications

1. Composant d'appui (2) destiné à être utilisé dans une prothèse humérale, le composant d'appui comprenant
une surface d'appui concave (4) qui se termine au niveau d'un rebord continu sensiblement circulaire (10), le rebord définissant un premier plan (12) ;
une surface de mise en prise convexe (6), qui se trouve sur un côté opposé du composant d'appui par rapport à la surface d'appui et se termine en un épaulement annulaire (18), l'épaulement définissant un second plan (14) ; et
une région de liaison (8) qui s'étend entre les premier et second plans, dans lequel les premier et second plans ne sont pas parallèles,
dans lequel le composant d'appui conjointement avec une tige (30) est conçu pour former une partie humérale d'une prothèse d'épaule inverse.

2. Composant d'appui (2) selon la revendication 1, dans lequel la surface d'appui concave (4) comprend moins d'un hémisphère.

3. Composant d'appui (2) selon la revendication 1 ou 2, dans lequel un angle entre les premier et second plans est compris entre 5 et 20 degrés.

4. Composant d'appui (2) selon l'une quelconque des revendications précédentes, dans lequel la région de liaison (8) comprend une paroi de liaison (22), sensiblement sous la forme d'un cylindre tronqué.

5. Composant d'appui (2) selon l'une quelconque des revendications précédentes, dans lequel l'épaulement annulaire (18) définit une surface d'appui annulaire (20).

6. Composant d'appui selon l'une quelconque des revendications précédentes, dans lequel l'épaulement annulaire comprend au moins un évidement.

7. Composant d'appui (2) selon l'une quelconque des revendications précédentes, où le composant d'appui est formé d'un matériau polymère.

8. Composant d'appui (2) selon la revendication 7, dans lequel le matériau polymère est du polyéthylène à masse moléculaire ultra-élevée.

9. Composant d'appui (2) selon l'une quelconque des revendications précédentes, comprenant en outre un composant de revêtement.

10. Composant d'appui (2) selon la revendication 9, dans lequel le composant de revêtement comprend une enveloppe métallique qui revêt la surface d'appui concave (4) du composant d'appui.

11. Prothèse humérale comprenant un composant d'appui (2) selon l'une quelconque des revendications précédentes et une tige (30).

12. Prothèse humérale selon la revendication 11, dans laquelle la surface de mise en prise (6) du composant d'appui (2) est reçue à l'intérieur de la tige (30) de telle sorte que la surface d'appui annulaire (20) du composant d'appui vient en prise avec une surface d'appui annulaire correspondante (36) sur la tige.

13. Prothèse humérale selon la revendication 11 ou 12, dans laquelle la surface d'appui annulaire (36) de la tige (30) comprend au moins un ergot faisant saillie (38), adapté pour être reçu à l'intérieur des évidements (24) sur l'épaulement annulaire (18) du composant d'appui (2).

14. Prothèse humérale selon l'une quelconque des revendications 11 à 13, dans laquelle l'épaulement annulaire du composant d'appui comprend une pluralité d'évidements disposés de façon circonférentielle autour du composant d'appui.

15. Prothèse humérale selon la revendication 14, dans laquelle la surface d'appui annulaire (36) de la tige (30) comprend une pluralité d'ergots faisant saillie (38), adaptés pour être reçus à l'intérieur de la pluralité d'évidements (24) sur l'épaulement annulaire (18) du composant d'appui (2).

16. Prothèse d'épaule (50) comprenant une prothèse humérale selon l'une quelconque des revendications 11 à 15 et une prothèse glénoïdienne (40).

17. Prothèse d'épaule (50) selon la revendication 16, dans laquelle la prothèse glénoïdienne (40) comprend une vis glénoïdienne (42) et une tête glénoïdienne (48).
